# NEW EUROPEAN PATENT SPECIFICATION

(11) **EP 1 138 294 B2**
(45) Date of publication and mention of the opposition decision: **11.06.2008**
(45) Mention of the grant of the patent: 20.10.2004
(21) Application number: 00117559.5
(22) Date of filing: 15.08.2000
(51) Int. Cl.: A61F 13/15, A61F 13/56

(54) **Thong pantiliner with flaps**
Einlage für Tangaslip mit Seitenflügel
Protège-culotte à utiliser avec une culotte tange avec des ailes de côté

(30) Priority: 29.03.2000 US 538010
(43) Date of publication of application: 04.10.2001
(73) Proprietor: McNEIL-PPC, Inc., Skillman NJ 08558 (US)
(72) Inventor: Neumann de Camacho, Maria Monika, 9-95 Cali, ID 88798 (CO); Ribeiro de Carvalho, Antonio Carlos, Sao Paulo, CPF 831,477.723-53 (BR); Haarer, Jutta S., Lawrence Ville NJ 08648 (US); Luzano, James, Elizabeth NJ 07208 (US); Passos Proglhof, Igor Philip, Sao Paulo CPF 042.551.898-11 (BR)
(74) Representative: Metten, Karl-Heinz

(56) References cited:
- WO-A-00/72790
- WO-A-98/25561
- US-A- 4 608 047
- US-A- 5 358 499
- US-A- 5 713 886
- US-D- 394 503

## Description

The invention relates to a pantiliner for use with thong underwear. It includes a pair of flaps extending laterally outward from each side of the posterior portion of the pantiliner, at least one of said flaps being adjacent to the posterior end of the pantiliner.

### Background of the Invention

Recently, the popularity of thong underwear has increased, giving rise to the development of pantiliners specifically for use with such underwear. Thong underwear presents unique design requirements for a pantiliner. Generally, thong underwear has a crotch portion that narrows to a minimal width of material in the rear of the undergarment, in order to expose all or a significant portion of the buttocks. For purposes of the present disclosure, the term "thong underwear" includes thongs, G-strings, Rio cut underwear, Brazilian cut underwear and the like.

U.S. Patent No. 5,713,886 to Sturino discloses a sanitary napkin or pantiliner for use with thong underwear. The pantiliner includes an absorbent core with first and second portions. The opposed sides of the second portion flare continuously from the sides of the first portion so that the second portion has, at all points along its length, a transverse width greater than that of the first portion. The '886 pantiliner has three flaps extending laterally from its sides. Two flaps extend from the sides of the second portion, while one flap extends from one side of the narrow, first portion.

U.S. Patent No. Des. 394,503 and UK Design Registration No. 2076491 both in the name of Perrini relate to designs for thong pantiliners having overall shapes similar to a light bulb with side flaps extending from the narrow portions of the pantiliners. In each design, the side flaps are located on the narrow portion of the pantiliner adjacent to where the narrow portion joins the wider portion of the pantiliner.

Similarly, UK Design Registration No. 2087071 also in the name of Perrini shows a thong pantiliner having a more gradual change in width along its length, also with side flaps. In this case, side flaps are located roughly in the middle of the pantiliner.

The patent document WO 00 72 790 A, relevant under Art. 54 (3) EPC, discloses an absorbent article having an anterior portion which is wider compared to the posterior end. On both long sides of the posterior portion there are arranged fastening tabs in form of separated, discrete pieces of material on the article.

Unfortunately, one problem with pantiliners of the sort described above is their ability to stay in place in thong underwear. First, the narrow posterior end of such pantiliners has little fabric to which to attach. Second, the area between the buttocks is sensitive and subject to a high degree of movement. Accordingly, the need exists for a thong pantiliner that will attach strongly to the thong underwear and stay there, so as not to reveal its presence or irritate the wearer.

With these factors in mind, applicants have discovered that a highly useful thong pantiliner with a pair of flaps may be made by placing at least one of the flaps adjacent the narrow, posterior end of the pantiliner. Such a pantiliner stays in place within thong underwear, thereby resulting in improved discretion and comfort for the wearer.

### Summary of the Invention

The invention provides a pantiliner according to claim 1.

### Brief Description of the Drawings

Figures 1-3 are plan views of thong pantiliners according to examples helpful for understanding the invention, but not covered by the invention having various shaped flaps.
Figure 4 is a plan view of a thong pantiliner according to an example helpful for understanding the invention, but not covered by the invention having staggered flaps.
Figure 5 is a plan view of a thong pantiliner according to the invention having connected flaps that extend continuously around the posterior end of the pantiliner.
Figure 6 is a plan view of a thong pantiliner according to the invention in which the anterior portion is triangular and the flaps are of an irregular shape.
Figure 7 is a plan view of a thong pantiliner according to the invention in which the midsection is biconcave in shape.
Figure 8 is a plan view of a thong pantiliner according to an example helpful for understanding the invention, but not covered by the invention in which the anterior portion is round and the midsection is of a uniform width.

### Detailed Description of the Invention

The pantiliner comprises a body portion comprising as layers an absorbent core and a backsheet underlying the absorbent core, that is, on the garment-facing side of the absorbent core. The absorbent core may comprise one or more than one layer of absorbent material, such as cellulose fibers, including wood pulp, regenerated cellulose fibers, cotton fibers, polyethylene fibers, polypropylene fibers, polyester fibers, bicomponent fibers, acrylic fibers, polyvinyl alcohol fibers, peat moss and superabsorbent polymers. Such fibers can be thermobonded, latex bonded, or a combination of the two, or bonded and densified in any manner known in the art. Any one or combinations of these absorbent materials may be used to make the absorbent core.

The backsheet is substantially or completely impermeable to liquids, and its exterior forms the garment-facing surface of the pantiliner. The backsheet may comprise any thin, flexible, body fluid impermeable material such as a polymeric film or other nonwoven comprising for example polyethylene, polypropylene, or cellophane. Alternatively, the backsheet may be a normally fluid permeable material that has been treated to be impermeable, such as impregnated fluid repellent paper or non-woven fabric material, or a flexible foam, such as polyurethane or cross-linked polyethylene. The thickness of the backsheet when formed from a polymeric film typically is about 0.005 to 0.002 inch.

The backsheet may be breathable, i.e., made of a film that is a barrier to liquids but permits gases to transpire. Known materials for this purpose include nonwoven materials and microporous films in which microporosity is created by stretching an oriented film. Single or multiple layers of permeable films, fabrics, and combinations thereof that provide a tortuous path, and/or whose surface characteristics provide a liquid surface repellent to the penetration of liquids may also be used to provide a breathable backsheet.

The pantiliner may optionally comprise a cover overlaying the absorbent core, that is on the body-facing side of the pantiliner. The cover may be formed from any fluid pervious material that is comfortable against the skin and permits fluid to penetrate to the absorbent core, which retains the fluid. The cover should retain little or no fluid to provide a relatively dry surface. A variety of cover materials are known in the art, and any of these may be used. For instance, the cover may be a fibrous non-woven fabric made of fibers or filaments of polymers such as polyethylene, polypropylene, polyester, bicomponent fibers, or cellulose. Alternatively, the cover may be formed from an apertured polymeric film. The thickness of the cover may vary from approximately 0.001 to 0.062 inch, depending on the material chosen.

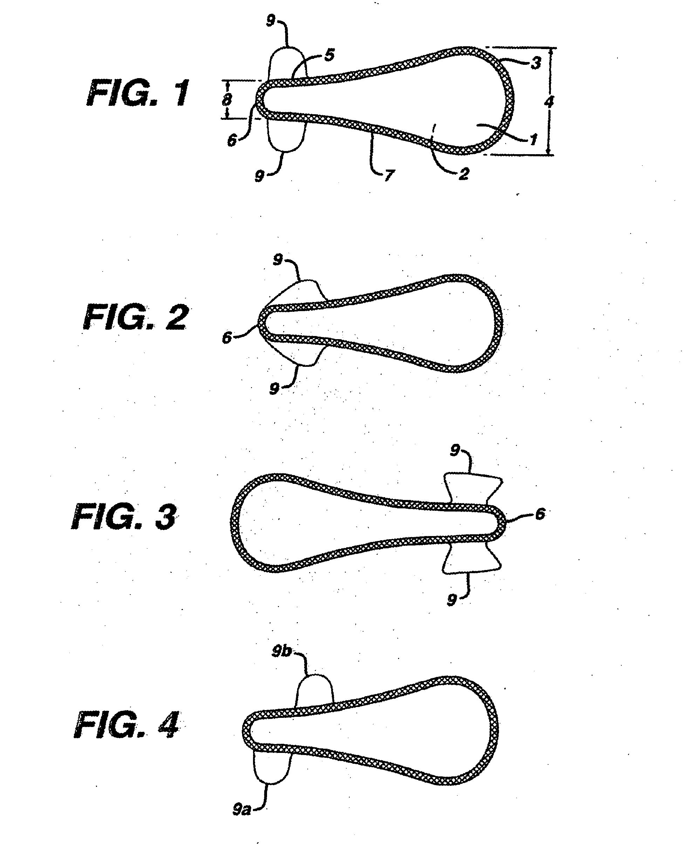

Figure 1 depicts a thong pantiliner according to an example helpful for understanding the invention, but not covered by the invention. The overall shape of the pantiliner is such that it will fit comfortably within the confines of thong underwear. Referring to Figure 1, the pantiliner comprises a midsection 7 connecting a relatively wide anterior portion 3 with a relatively narrow posterior portion 5. In particular, the maximum width 4 of the anterior portion 3 of the pantiliner is greater than the width 8 of the posterior end 6 of the pantiliner. Figure 1 also depicts the absorbent core 1 of the pantiliner on the body-facing surface of the pantiliner and the backsheet 2 underneath.

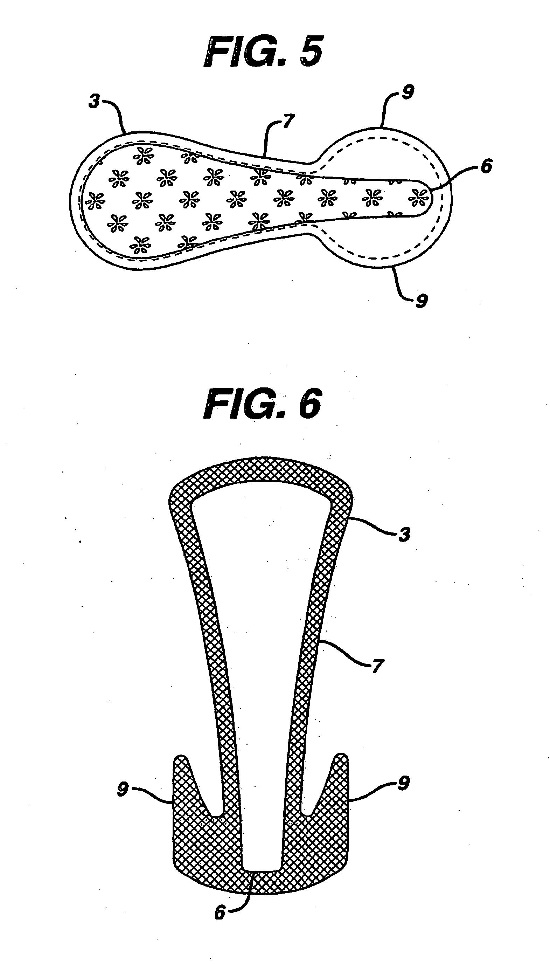

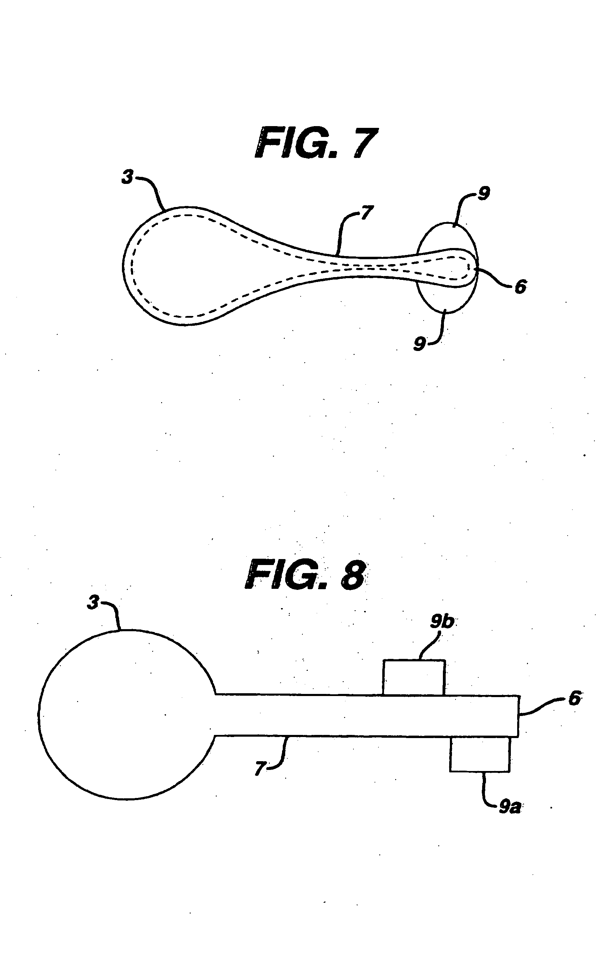

The precise shapes of the anterior portion, midsection, and the posterior portion may vary as desired, so long as the maximum width of the anterior portion is greater than the width of the posterior end. For example, the anterior portion may have the overall shape of a bulb, as shown in Figures 1-5. Alternatively, the anterior portion may have the overall shape of a triangle as shown in Figure 6, or may be round as shown in Figure 8. The midsection may be tapered and narrow at a substantially continuous rate along its length, as shown in Figures 1-6. The midsection may be biconcave in shape, as shown in Figure 7. The midsection may also be of a narrow, uniform width like a stem, as shown in Figure 8. This embodiment may be particularly useful in G-string underwear.

The overall dimensions of the pantiliner are preferably as follows. The length is preferably in the range of about 5 to 8 inches. The maximum width of the anterior portion is preferably in the range of about 2 to 3 inches. The thickness of the pantiliner is preferably in the range of about 0.04 to 0.2 inches.

Referring again to Figure 1, flaps 9 extend from each side edge of the posterior portion 5 of the pantiliner. Flaps, also known variously as wings, tabs, and the like, are generally speaking flexible and configured to be folded over the edges of the underwear so that the flaps are disposed between the edges of the underwear and, in this case, the wearer's buttocks. According to the present invention, at least one of the flaps 9 is adjacent to the posterior end 5 itself. In Figure 1, both flaps 9 are adjacent the posterior end 5. In this manner, one or both flaps securely anchor the posterior end to the thong underwear.

A flap is adjacent to the posterior end of the pantiliner when the distance between the posterior end and the flap edge closest to the posterior end is no more than 10%, preferably no more than 8%, of the total length of the pantiliner.

Figures 5, 6 and 7 show pantiliners according to the invention with both flaps 9 adjacent to the posterior end 6 of the pantiliner. In use, these flaps are folded over the edge of the thong underwear and, depending on their size and shape, may overlap one another on the reverse side of the underwear.

Figures 4 and 8 shows pantiliners according to examples helpful for understanding but not covered by the invention in which the flaps 9 are staggered. One of the flaps 9a is adjacent the posterior end 6 of the pantiliner. The other flap 9b is positioned somewhat remote from the posterior end 6. In use, these flaps are also folded over the edge of the thong underwear, but do not overlap one another on the reverse side of the underwear. This has the dual advantages of minimizing the thickness of material between the buttocks, as well as fixing a longer portion of the pantiliner more securely to the underwear. It is also possible, of course, to design the flaps so that they overlap one another only partially.

The flaps are made from an extension of the backsheet. It is preferred in any case that the flaps have a liquid impervious barrier sheet to prevent exudates which reach the flaps from soiling the edges of the wearer's underwear.

If desired, the flaps may optionally be provided with a thin layer of absorbent material. Theoretically, only a relatively small amount of discharged fluid should reach the flaps, therefore, only a relatively small amount of absorbent material is needed. Such absorbent material may be the same as or different from the absorbent material used to make the absorbent core. For example, the absorbent core may simply extend into the flaps. Any absorbent material present in the flaps should be relatively flexible.

The flaps may be regular or irregular, symmetric or asymmetric in shape.

The flaps 9 shown in Figure 5 are connected to each other and extend continuously around the posterior end 6 of the pantiliner. In this embodiment of the invention, the flaps may be an extension of a laminate of the cover and the backsheet, as described above.

U.S. Pat. No. 4,687,478 to Van Tilburg; U.S. Pat. No. 4,589,876 also to Van Tilburg, U.S. Patent No. 4,900,320 to McCoy, and U.S. Pat No. 4,608,047 to Mattingly relate to the use of flaps in sanitary protection articles. The disclosures of these patents are incorporated herein by reference in their entirety.

The pantiliner may be applied to the crotch of thong underwear by placing the garment-facing surface of the pantiliner against the inside surface of the crotch of the thong underwear. Strips of positioning adhesive may be applied to the garment-facing surface of the pantiliner and flaps to help maintain them in place. Preferably, a pressure-sensitive adhesive is used for this purpose, which refers to any releasable adhesive or releasable tenacious means. Suitable pressure sensitive adhesives include for example water-based adhesives such as acrylate adhesives. Alternatively, the adhesive may comprise "hot melt" rubber adhesives or two-sided adhesive tape. Suitable positioning adhesives are commercially available from H.B. Fuller Company and ATO Findley. The positioning adhesive may be applied to the garment-facing surface of the pantiliner by any means known in the art. such as slot coating, transfer coating, control coating, and the like.

A paper release strip that has been coated on one side may be applied to protect the adhesive prior to use. The coating, for example silicone. reduces adherence of the coated side of the release strip to the adhesive. The release strip can be formed from any suitable sheet-like material which, when coated, adheres with sufficient tenacity to the adhesive to remain in place prior to use but can be readily removed when the pantiliner is to be used.

Prior to use, the flaps may be left open or folded on top or underneath the pantiliner in any one of a variety of configurations. A single release strip may be used for both the flaps and the garment-facing surface of the pantiliner, or two or more separate release strips can be employed.

The pantiliner may comprise other known materials, layers, and additives, such as transfer layers, foam layers, net-like layers, perfumes, medicaments, moisturizers, odor control agents, and the like, many examples of which are known in the art. The pantiliner can optionally be embossed with decorative designs using conventional techniques.

The following non-limiting example further illustrates the invention.

### Example

A thong pantiliner according to the invention was made as follows. The pantiliner comprised a nonwoven cover made from polypropylene fibers, an absorbent core comprising pulp and bicomponent fibers, and a polyethylene film backsheet. These layers were held together with lamination adhesive commercially available from H.B. Fuller Company. Positioning adhesive, also commercially available from H.B. Fuller, was applied to the garment-facing surface of the backsheet, and covered by silicone-coated release paper.

The pantiliner had the shape shown in Figure 5. It comprised flaps extending continuously around and connected to one another along the posterior end of the pantiliner.

## Claims

1. A pantiliner comprising an absorbent core (1) and a backsheet (2) underlying the absorbent core (1), said pantiliner including an anterior portion (3) having a maximum width (4), a posterior portion (5) adjacent a posterior end (6) of said pantiliner, and a midsection connecting the anterior and posterior portions (3;5), wherein the width (8) of the posterior end (6) of said pantiliner is smaller than the maximum width (4) of the anterior portion (3), said pantiliner having at least one flap (9) being adjacent to the posterior end (6), the flap (9) is made of an extension of the backsheet (2) **characterized in that** a flap (9) extends from each side of the posterior portion (5), wherein the flaps (9) extend continuously around and are connected to one another along the posterior end (6) of the pantiliner.

2. The pantiliner of claim 1, wherein the anterior portion (3) is bulb-shaped.

3. The pantiliner of claim 1, wherein the anterior portion (3) is triangular.

4. The pantiliner of claim 1, wherein the midsection is biconcave in shape.

5. The pantiliner of claim 1, wherein the midsection is tapered.

6. The pantiliner of claim 1 further comprising a cover.

7. The pantiliner of claim 1, wherein the absorbent core (1) comprises absorbent material selected from the group consisting of pulp fibers, bicomponent fibers, polyester fibers, and superabsorbent polymers.

8. The pantiliner of claim 1 comprising a breathable backsheet (2).

## Patentansprüche

1. Slipeinlage mit einem absorbierenden Kern (1) und einer rückwärtigen Schicht (2), die unter dem absorbierenden Kern (1) liegt, wobei die Slipeinlage einen vorderen Abschnitt (3) mit einer maximalen Breite (4), einen hinteren Abschnitt (5), der an ein hinteres Ende (6) der Slipeinlage angrenzt, und einen Mittelbereich aufweist, welcher den vorderen und hinteren Abschnitt (3; 5) verbindet, wobei die Breite (8) des hinteren Endes (6) der Slipeinlage kleiner als die maximale Breite (4) des vorderen Abschnitts (3) ist, die Slipeinlage mindestens eine Lasche (9) aufweist, die dem hinteren Ende (6) benachbart ist, wobei die Lasche (9) durch eine Erweiterung der rückwärtigen Schicht (2) gebildet wird, **dadurch gekennzeichnet, daß** sich eine Lasche (9) von jeder Seite des hinteren Abschnitts (5) erstreckt, wobei die Laschen (9) sich kontinuierlich um das hintere Ende (6) der Slipeinlage erstrecken und miteinander entlang des hinteren Endes (6) der Slipeinlage verbunden sind.

2. Slipeinlage nach Anspruch 1, bei welcher der vordere Abschnitt (3) birnenförmig ist.

3. Slipeinlage nach Anspruch 1, bei welcher der vordere Abschnitt (3) dreieckig ist.

4. Slipeinlage nach Anspruch 1, bei welcher der Mittelbereich eine bikonkave Form aufweist.

5. Slipeinlage nach Anspruch 1, bei welcher sich der Mittelbereich verjüngt.

6. Slipeinlage nach Anspruch 1, welche weiterhin eine Abdeckung umfaßt.

7. Slipeinlage nach Anspruch 1, bei welcher der absorbierende Kern (1) ein absorbierendes Material aufweist, welches aus der Gruppe ausgewählt ist, die aus Pulpfasern, Bikomponentenfasem, Polyesterfasern und superabsorbierenden Polymeren besteht.

8. Slipeinlage nach Anspruch 1, welche eine atmungsaktive rückwärtige Schicht (2) aufweist.

## Revendications

1. Protège-slip comprenant un coeur absorbant (1) et une couche inférieure (2) située sous le coeur absorbant (1), ledit protège-slip comprenant une partie antérieure (3) possédant une largeur maximale (4), une partie postérieure (5) adjacente à une extrémité postérieure (6) dudit protège-slip, et une section intermédiaire reliant les parties antérieure et postérieure (3 ; 5), dans lequel la largeur (8) de l'extrémité postérieure (6) dudit protège-slip est plus petite que la largeur maximale (4) de la partie antérieure (3), ledit protège-slip possédant au moins une ailette (9) adjacente à l'extrémité postérieure (6), l'ailette (9) étant formée d'une extension de la couche inférieure (2), **caractérisé en ce qu'**une ailette (9) s'étend de chaque côté de la partie postérieure (5) et les ailettes (9) s'étendent de façon continue tout autour et sont reliées l'une avec l'autre le long de l'extrémité postérieure (6) du protège-slip.

2. Protège-slip selon la revendication 1, dans lequel la partie antérieure (3) a la forme d'un bulbe.

3. Protège-slip selon la revendication 1, dans lequel la partie antérieure (3) est triangulaire.

4. Protège-slip selon la revendication 1, dans lequel la section intermédiaire a une forme biconcave.

5. Protège-slip selon la revendication 1, dans lequel la section intermédiaire est effilée.

6. Protège-slip selon la revendication 1 comprenant en outre un sachet.

7. Protège-slip selon la revendication 1, dans lequel le coeur absorbant (1) comprend un matériau absorbant sélectionné dans le groupe composé des fibres de pâte à papier, des fibres bicomposées, des fibres de polyester, et de polymères superabsorbants.

8. Protège-slip selon la revendication 1 comprenant une couche inférieure respirante (5).
